Europäisches Patentamt

**European Patent Office**

Office européen des brevets

(11) Publication number: **0 418 975 A1**

## EUROPEAN PATENT APPLICATION

(21) Application number: **90202474.4**

(51) Int. Cl.5: **C07C 2/84**

(22) Date of filing: **18.09.90**

(30) Priority: **19.09.89 US 409375**

(43) Date of publication of application:
**27.03.91 Bulletin 91/13**

(84) Designated Contracting States:
**AT BE CH DE DK ES FR GB GR IT LI LU NL SE**

(71) Applicant: **UNION CARBIDE CHEMICALS AND PLASTICS COMPANY, INC.**
**39 Old Ridgebury Road**
**Danbury Connecticut 06817-0001(US)**

(72) Inventor: **Campbell, Kenneth Dwight**
**101 Bowhunter Road**
**Charleston, West Virginia 25314(US)**

(74) Representative: **Smulders, Theodorus A.H.J., Ir. et al**
**Vereenigde Octrooibureaux Nieuwe Parklaan 107**
**NL-2587 BP 's-Gravenhage(NL)**

(54) Low temperature catalysts for oxidative coupling processes.

(57) Oxidative coupling of lower alkane to higher hydrocarbon is conducted using supported catalyst comprising an activity promoting amount of at least one cobalt, nickel or rhodium compound and at least one catalytically active Group IA and Group IIA metal compound in the presence of a halogen-containing component.

## LOW TEMPERATURE CATALYSTS FOR OXIDATIVE COUPLING PROCESSES

This invention was made under United States of America Government support under Contract No. DE-AC22-87PC79817 awarded by the Department of Energy. The Government has certain rights in this invention.

This invention relates to low temperature catalytic processes for the oxidative coupling of lower molecular weight alkane to higher molecular weight hydrocarbons.

Background of the Invention

Processes for the conversion of lower molecular weight alkanes such as methane to higher molecular weight hydrocarbons which have greater value are sought. One of the proposals for the conversion of lower molecular weight alkanes is by oxidative coupling. For instance G. E. Keller and M. M. Bhasin disclose in Journal of Catalysis , Volume 73, pages 9 to 19 (1982) that methane can be converted to, e.g., ethylene. The publication by Keller, et al. has preceded the advent of substantial patent and open literature disclosures by numerous researchers pertaining to processes for the oxidative coupling of lower alkanes and catalysts for such processes.

In order for an oxidative coupling process to be commercially attractive, the process should be capable of providing a good rate of conversion of the lower alkanes with high selectivity to the sought higher molecular weight hydrocarbons. Since conversion and selectivity can be enhanced by catalysts, catalytic processes have been the thrust of work done by researchers in oxidative coupling.

Even using catalysts, high temperatures have been required to achieve desirable conversions of hydrocarbons in oxidative coupling processes. The high temperatures used in oxidative coupling, often in excess of $700^\circ$ C, require special materials of construction, cause catalyst stability problems, and enhance the unselective oxidation of the reactants and products. Accordingly, catalysts are sought that exhibit enhanced activities without deleterious effects on the selectivities to higher hydrocarbons.

Numerous catalysts have been proposed by researchers for oxidative coupling processes. These catalysts have included catalysts containing alkali and/or alkaline earth metals. The alkali and alkaline earth metals have been suggested as being in the oxide, carbonate and halide forms. Other components such as rhenium, tungsten, copper, bismuth, lead, tin, iron, nickel, zinc, indium, vanadium, uranium, osmium, zirconium, titanium, lanthanum, aluminum, chromium, cobalt, beryllium, germanium, antimony, gallium, manganese, yttrium, cerium, praseodymium and other rare earth oxides, scandium, molybdenum, thallium, thorium, cadmium, boron, among other components, have also been suggested for use in oxidative coupling catalysts. See, for instance, United States Patents Nos. 4,450,310; 4,443,646; 4,499,324; 4,443,654; 4,443,618; 4,523,050; 4,442,647; 4,499,323; 4,443,644; 4,444,981; 4,695,668; 4,704,487; 4,777,313; 4,780,449; International Patent Publication WO 86/07351, European Patent Applications 189079 (1986); 206042 (1986); 206044 (1986), and 177327 (1985), Australian Patent No. 52925 (1986), Moriyama, et al., "Oxidative Dimerization of Methane Over Promoted MgO: Important Factors," Chem. Soc. Japan, Chem. Lett, 1165 (1986), and Emesh, et al., "Oxidative Coupling of Methane over the Oxides of Groups IIIA, IVA and VA Metals," J. Phys. Chem, Vol. 90, 4785 (1986).

Several researchers have proposed the use of alkali or alkaline earth metals in the form of halides (e.g., chloride, bromide or iodide) in oxidative coupling catalysts. Australian Patent No. 52925 discloses the use of supported calcium chloride, barium bromide, potassium iodide, lithium chloride, cesium chloride, among others for catalysts to oxidatively couple methane to ethane and ethylene. The patentees disclose feeding hydrogen halide to the reaction zone. European Patent Application 210 383 (1986) discloses the addition of gas phase material containing halogen component such as chlorine, methyl chloride and methyl dichloride. Enhanced selectivities are reported when the halogen component is present. The catalysts include those containing one or more of alkali and alkaline earth metal, alkali metal with lanthanide oxide, zinc oxide, titanium oxide or zirconium oxide, and others. United States Patent No.4,654,460 discloses the addition of a halogen-containing material either in the catalyst or via a feed gas in an oxidative coupling process. rne catalyst contains one or more alkali metal and alkaline earth metal components. Although no working examples are provided, conversions of methane are said to be increased with halides and selectivities to higher hydrocarbons, particularly ethylene, improved. See also, Burch, et al., "Role of Chlorine in Improving Selectivity in the Oxidative Coupling of Methane to Ethylene," Appl. Catal., Vol. 46, 69 (1989) and "The Importance of Heterogeneous and Homogeneous Reactions in Oxidative Coupling of Methane Over Chloride Promoted Oxide Catalysts," Catal. Lett., Vol 2, 249 (1989), and Minachev, et al., "Oxidative Condensation of Methane," Russ. Chem. Rev., Vol. 57, 221 (1988).

Group VIII metals have been proposed as components in oxidative coupling catalysts, but their potential has been severely limited. Keller, et al., supra, evaluated numerous metal components for oxidative coupling in a pulsed mode system. They concluded in Figure 6 that iron, nickel, copper, silver and platinum have, in the pulsed system, no activity above that of bare support and that cobalt had possibly small activity above that of bare support.

Mitchell, et al., in United States Patents Nos. 4,172,810; 4,205,194 and 4,239,658 propose multicomponent catalysts containing a Group VIII noble metal having a molecular weight of 45 or greater, nickel or a Group 1B noble metal having an atomic number of 47 or greater; a Group VIB metal oxide and a Group IIA metal component on a support for methane coupling via a sequential process. They propose that the catalyst can further contain, inter alia , iron, cobalt or a metal of the actinide or lanthanide series. They opine that Group VIII noble metal, nickel or Group 1B noble metal would dissociatively chemisorb methane; Group VIB reducible metal oxides would be reduced by adsorbed hydrogen and thus produce water, and Group IIA metal oxides would convert the adsorbed methane to carbides. The postulated carbides were stated by the patentees to be intermediates in the formation of aromatic compounds. The catalysts are described as being supported on a refractory support such as alumina. The catalyst is disclosed as being operated in a sequential (or pulsed) mode in which the oxygen containing gases and methane containing gases are alternatively cycled to the reaction zone. The catalyst becomes coked with use and therefore requires periodic regeneration.

Garcia, et al., "Direct Catalytic Synthesis of Ethylene from Methane", React., Kinet, Catal. Lett., Vol. 28, 481 (1985), disclose the use of, e.g., platinum and cobalt containing catalysts for oxidative coupling. The authors operated in the sequential mode and noted that long induction periods are required between detecting higher hydrocarbon products.

"It is interesting to note the long induction periods required for achieving detectable conversions. ". . . "This would indicate that ehtylene (sic) production can only occur when a significant surface carbon concentration is reached." (p. 434). The role of Co is postulated to ". . . chemisorb $CH_4$ dissociatively and provide additional surface species," (p. 435) and play "...the role of oxygen donor..." (p.435).

The sequential process for oxidative coupling provides numerous disadvantages for commercial production of large volume, commodity chemicals such as ethylene. For instance, the process is stoichiometric and hence large volumes of catalyst are required to sorb sufficient oxygen for the coupling, fluid bed or cyclic processing equipment must be used and is subject to greater wear and maintenance than normal fixed bed processes, and the processes tend to form heavy products including carbonaceous deposits which require periodic regenerations to remove. Accordingly, processes are sought which are operable in the cofeed mode of operation, i.e., the feed gas to the reaction zone comprises both the alkane and oxygen for the oxidative coupling reaction.

A number of workers have therefore attempted to provide catalysts operable in the cofeed mode. In many of the reported catalysts using cobalt and nickel, these components are present in very large amounts. For instance, United States Patent No. 4,620,057 reports the use of Co/Zr/S/P/Na/K/Cl/O for oxidative coupling of methane. The major constituents in the catalyst were Co and S and the cobalt is preferably present as cobalt sulfide (Col. 8, lines 7 and 8). Chloride addition is said to improve both the conversion of methane and the $C_2$ selectivity; however the material is capable of converting methane to higher hydrocarbons in the absence of halide. If the catalyst were prepared without halogen and a gas containing halogen is co-fed with the feed material during the reaction, then the presence of potassium in the contact material is reported to be necessary (Col. 5 lines 41 to 45). Certain components of the contact material were considered a necessity (cobalt; a metal selected from the group consisting of Zr, Zn, Nh, In, Pb and Bi; phosphorus; at least one Group IA metal and oxygen). See also, European Patent Application No. 210,383 (1987) which notes that a cobalt-based catalyst

"... does appear to be affected by the degree of oxidation thereof. It has been found that after a period of use, in the presence of a free oxygen containing gas, there is a tendency for this contact material to become 'overoxidized' and lose its reaction-promoting activity." (p. 13-14).

The European Patent Application discloses a regeneration step to deplete the overoxidation. A reducing gas is passed over the catalyst bed to effect the regeneration. This regeneration is said to be best accomplished by simultaneous contact with a halogen or immediately after contact with a halogen.

Otsuka, et al., "Active and Selective Catalysts in the Oxidative Coupling of Methane: Nickel Oxide with Alkali Metals," Inorg. Chim. Acta., Vol. 118, L23 (1986)and "Synthesis of Ethylene by Partial Oxidation of Methane Over Transition Metal Oxides with Alkali-Chlorides," Studies in Surface Science and Catalysis: #36, Methane Conversion Symposium, Auckland, New Zealand, 383 (1987) report the use of alkali doped NiO as methane conversion catalysts. The addition of LiCl, LiBr, $LiNO_3$, or $Li_2CO_3$ to NiO (20 mole% alkali)

resulted in high $C_2$ selectivities; however, the halide materials gave much higher ethylene/ethane ratios. $C_2$ yields between 6 and 15% are reported.

A number of workers have compared these cobalt and nickel-based catalysts to other catalyst containing, iron, copper, zinc, etc.

Otsuka, et al., "Synthesis of Ethylene by Partial Oxidation of Methane Over Oxides of Transition Metals with LiCl", Chem. Lett. 903 (1986), and "Synthesis of Ethylene by Partial Oxidation of Methane Over Transition Metal Oxides with Alkali-Chlorides," Studies in Surface Science and Catalysis: #36, Methane Conversion symposium, Auckland, New Zealand, 383 (1987), report the use of lithium chloride doped Ti, Cr, Mn, Fe, Co, Ni, Cu and Zn oxides as methane conversion catalysts. Without LiCl, the materials tended to favor alkane combustion. The highest $C_2$ yields were obtained with LiCl doped Mn or Ni-oxides. LiCl-doped cobalt, copper and zinc oxides also provided $C_2$'s while iron oxide did not. The use of NaCl instead of LiCl with Mn-oxide was found to give better results. (Otsuka, et al., "Active Catalysts in Oxidative Coupling of Methane," J. Chem. Soc., Chem. Commun., 388(1987)).

Otsuka, et al., "Alkali Metal Doped Transition Metal Oxides Active for Oxidative Coupling of Methane," Inorg. Chem. Acta, Vol. 146, 243 (1988), report the use of alkali metal-doped transition metals as coupling catalysts. Oxides of Cr, Mn, Fe, Co, Ni, and Cu were doped with alkali nitrates. The highest $C_2$ selectivity was reported for $LiNO_3/NiO$ (46.9%). The active phase for the $LiNO_3/NiO$ catalyst was postulated as $LiNiO_2$. $LiNO_3/Fe_2O_3$ and $LiNO_3/Co_2O_3$ catalysts provided comparable selectivities of about 2 to 3 percent, and $LiNO_3/CuO$ provided a slightly higher selectivity. When $NaNO_3$ and $KNO_3$ were used as the dopant $Co_2O_3$ was better than CuO which was better than $Fe_2O_3$ in selectivities to $C_2$'s.

Several workers have published on the use of, e.g., cobalt, copper, silver, platinum, nickel and iron, as promoters in oxidative coupling catalysts wherein these compounds are present in relatively small amounts.

Larkin and Nordin, "Oxidative Dehydrogenation of Methane to Form Higher Hydrocarbons," Studies in Surface Science and Catalysis:#36, Methane Conversion Symposium, Auckland, New Zealand, p. 409 (1987), studied the effect of adding transition metals to Li/MgO methane oxidative coupling catalysts. The addition of two mole percent Zn, Cu, Ni, Co, Fe, Mn, or Or-oxide to Li/MgO increased the methane conversion at 800° C. Mn addition resulted in comparable activity at 750° C to the undoped catalyst at 800° C. Mn, Fe, and Co were most effective in terms of $C_2$ selectivity. Cu, Zn and Ni appeared to decrease selectivity to $C_2$'s in comparison to the undoped Li/MgO catalyst.

Ahmed, et al., "The Enhancement of the Oxidative Coupling of Methane on Oxide/$SiO_2$ Catalysts by Tetrachloromethane," Catal. Lett., Vol. 2, 309 (1989), report the use of carbon tetrachloride as a feed stream additive for oxidative coupling of methane using a normally 5 weight percent metal loading on silica catalyst. All of Ba, Cs, Mn, K, Ca, Co, Bi, Pb and Tl tended to increase the conversion of methane in the absence of carbon tetrachloride. With carbon tetrachloride being added to the feed stream the methane conversions with all but Pb and Tl increased and Co provided the highest conversion. The selectivities to $C_2$'s decrease for Co and Tl. (See Table 1 of the article.)

Garnett, et al., "Catalytic Oxidation of Methane over $AlPO_4$-5 and Metal Doped $AlPO_4$-5″, Studies in Surface Science and Catalysis: #36 Methane Conversion Symp., Auckland, New Zealand, April 1987, page 389, report the use of Pt, V, Co, Cu, Pb, and Ag on $AlPO_4$-5 molecular sieve. Pt-$AlPO_4$-5 and V-$ARPO_4$-5 showed greatly increased reactivity with dramatic decreases in $C_2$ selectivity. Cu-$AlPO_4$-5 and Co-$AlPO_4$-5 showed a slight changes from the $AlPO_4$-5 results. Pb-$AlPO_4$-5 and Ag-$AlPO_4$-5 showed both enhanced selectivity and reactivity.

Summary of the Invention

By this invention processes are provided for the oxidative coupling of lower alkane to produce heavier hydrocarbons which can occur at relatively low temperatures for oxidation coupling processes. In the processes of this invention, both the alkane and reactive oxygencontaining material are cofed to a reaction zone, thereby avoiding problems associated with sequential oxidative coupling processes. Moreover, with the low oxidative coupling temperatures that are achievable using the processes of this invention, enhanced catalyst stability can be obtained.

In accordance with this invention, supported catalysts are used which comprise at least one of cobalt, nickel, and rhodium in an amount up to about 2 weight percent based on the weight of the catalyst which amount is sufficient to provide enhanced oxidative coupling activity, say, at 600-650° C as compared to a similar catalyst but not containing any of the aforementioned metals under otherwise identical process conditions, and a sufficient amount of at least one alkali or alkaline earth metal to enhance the selectivity of the catalyst. The processes are conducted in the presence of halogen-containing component.

In the oxidation coupling processes of this invention, lower alkane, e.g., 1 to 3 carbon atoms, and reactive oxygen containing material are fed to a reaction zone containing a catalytically effective amount of oxidative coupling catalyst in the presence of halogen-containing component; the reaction zone is maintained under oxidative coupling conditions comprising a bulk temperature of less than about 700°C to convert at least a portion of the alkane to higher hydrocarbon; and an effluent comprising higher hydrocarbon produced in the reactor zone is withdrawn.

A halogen-containing component is provided in the reaction zone in an amount sufficient to enhance at least one of the selectivity of alkane conversion to heavier hydrocarbon and the activity. The halogen-containing component may be provided by a halogen-containing component in the catalyst and/or, preferably provided as an additive to the feed to the reaction zone. The halogen-containing component comprises at least one of chlorine, bromium and iodine, either in molecular form or in combined form.

The processes of this invention, even though a relatively small amount of cobalt, nickel and/or rhodium are present in the catalyst, are able to achieve substantially enhanced alkane conversion at lower bulk reaction temperatures. Moreover, in preferred aspects of the invention, the cobalt and/or nickel and/or rhodium is provided in an amount sufficient to enhance the ethylene to ethane mole ratio in the reactor zone effluent as compared to a similar catalyst which does not contain the aforementioned component under the same conditions. Thus, an advantage that can be provided by the processes of this invention is the ability to provide a desirably high mole ratio of ethylene to ethane, e.g., often 2.5:1 or more, at the lower oxidative coupling conditions of this invention.

Detailed Discussion of the Invention

In accordance with this invention, lower alkane is converted to higher hydrocarbons. The lower alkane preferably comprises at least one of methane, ethane and propane, and because of its abundance and the desire to convert it to higher hydrocarbons, methane is the most preferred component in the feed. The products of the conversion are higher hydrocarbons, especially alkanes and alkenes. Often, the desired conversion products are alkenes of two to four carbon atoms, especially ethylene and propylene. Because of its widespread use in commodity chemicals, product mixtures exhibiting a high selectivity to ethylene are typically preferred. The reaction is conducted in the presence of a reactive oxygen-containing material (oxidizing material) which for the purposes herein means atomic or molecular oxygen or a compound or chemical complex that contains an oxygen atom available for the oxidative coupling.

The hydrocarbon conversion process is conducted in a cofeed, or simultaneous process, in which both the oxidizing material and the alkane-containing feed are provided at the same time to the reaction zone.

In a cofeed process, the oxidizing material and alkane may be introduced by one or more separate streams or, most commonly, in a premixed stream. Generally, the mole ratio of alkane to active oxygen atom of the oxidizing material (an active oxygen atom is an oxygen atom that is available for oxidation) is at least about 1:2, say, about 1:2 to 50:1, preferably 1:1 to 20:1, The alkane typically comprises at least about 2 volume percent, e.g., up to about 95, say, 5 to 90, volume percent of the total gases fed to the reaction zone. Frequently, the feed streams are diluted with essentially inert gases such as helium, nitrogen, argon, steam, and carbon dioxide. When diluted, the diluent usually provides between about 5 to 95 volume percent of the feed streams.

The oxidizing material may be any suitable oxygen-bearing material which, under the conditions in the reaction zone, yields an active oxygen atom for the oxidative coupling. Convenient oxidizing materials are normally gaseous such as molecular oxygen, (e.g., as oxygen, enriched air or air), ozone and gases which yield oxygen such as $N_2O$. Materials that are liquid or solid at ambient conditions may also be used provided that they can be facilely introduced into the reaction zone.

The reaction proceeds at elevated temperatures. Generally, a minimum temperature must be achieved before significant higher hydrocarbon production occurs. If the temperature is too high, an undue amount of the hydrocarbon is consumed in oxidation or degradation reactions. The bulk temperature, i.e., temperature of the reaction zone as opposed to localized temperature which may exist in the reaction zone, is less than about 700°C. Offen, the temperature is in the range of about 500° to 700°C., and preferably about 550° to 675°C. The reactants are usually preheated prior to their introduction into the reaction zone; for instance, to within about 200°C, preferably about 100°C of the temperature in the reaction zone.

The pressure in the reaction zone may vary widely from less than atmospheric to 100 atmospheres absolute or more, say, 1 to 50, atmospheres absolute.

In general, the reactions proceed rapidly and, hence, the reactants may reside in the reaction zone under reaction conditions for a relatively short period of time, e.g., less than about 20 seconds, often less than about 10 seconds. Frequently, the residence time is about 0.001 to 5, say, 0.1 to 3, seconds. The gas hourly space velocity based on the total gases fed to the reaction zone to the volume of the reaction zone is often about 50 to 50,000, preferably, 500 to 15000, reciprocal hours. Since the alkane conversion reactions do not require the presence of catalyst to proceed, the overall volume of the vessel in which the reaction takes place may be substantially larger than that of the reaction zone containing catalyst. Even so, the volume of the reaction zone is frequently calculated as the volume of the vessel filled with catalyst.

The reaction may be conducted in any suitable reactor capable of providing the reaction temperatures.

The catalysts of this invention are generally characterized as supported, i.e., the cobalt and/or nickel and/or rhodium compound is dispersed on another material which provides the shape to the catalyst. The support, which is catalytically-acceptable, may be catalytically active or inert; however, the preferred supports usually exhibit some catalytic activity.

The support material may comprise refractory oxides, e.g., alumina, zirconia, titania, silica, spinels, perovskites (e.g., $ABO_3$ wherein A is a Group IIA metal and B is a Group IVA metal), aluminosilicates, alkaline earth oxides (e.g., magnesium oxide, calcium oxide, barium oxide and strontium oxide); alkaline earth carbonates (e.g., barium carbonate and strontium carbonate), and the like. Advantageously, the support material has a surface area of at least about 0.1, preferably, at least about 0.2, say, 0.2 to 60 or 100 or more, square meters per gram. (Determined by the nitrogen B.E.T. Method, J.Am.Chem. Soc., Vol. 60, 309 (1938)).

The total amount of cobalt and/or nickel and/or rhodium compound is frequently at least about 0.01, e.g., about 0.01 to 1, say, about 0.2 to 0.8, weight percent (calculated as the metal) based on the total weight of the catalyst. The cobalt, nickel or rhodium may be initially provided on the catalyst in elemental or combined form (e.g., as a nitrate, oxide, hydroxide, carbonate, chloride, etc.). While not wishing to be limited to theory, it is believed that the metal should be maintained in at least a partially chemically combined form (e.g., as in one or more of oxide, carbonate, hydroxide, and halide, especially a chloride, bromide or iodide) during the process. preferably, the catalysts comprise cobalt.

The catalysts preferably contain at least one Group IA or Group IIA compound. Most preferably, at least one Group IIA component, especially barium and/or strontium, is present. These components may be oxides, hydroxides, or salts, e.g., halides ($X^-$), oxyhalides ($OX^-$), halites ($XO_2^-$) halates ($XO_3^-$), perhalates ($XO_4^-$) (wherein X is one or more of chlorine, bromine and iodine), carbonates, sulfates, molybdates, tungstates, cerates, nitrates, etc. The Group IA and Group IIA components, if not contained in the support, are present in an amount of at least about 0.01, say, about 0.1 to 50, most preferably, at least about 2 to 30, weight percent of the total catalyst.

Other adjuvants that may be present in the catalyst include metal oxides, hydroxides and salts of one or more of Group IIIA (including lanthanide series) and Group IVA (e.g., titanium and zirconium) elements. These adjuvants may be used in amount of between 0.001 and 50 weight percent of the total catalyst.

The supported catalysts may be prepared by any convenient technique. Techniques which have been proposed include coating the catalyst support with a slurry or paste of the ingredients or impregnating the support using a solution or suspension or complex of the ingredients (the impregnation may be simultaneous for all components or sequential). The impregnation may be by an incipient wetness technique or by immersion in the mother liquor or by evaporation of a solvent from a solution or suspension combining the support. The catalysts may be dried and, optionally, calcined.

The catalyst size and configuration may vary depending upon the reactor type. For fluid, ebulating and riser reactors, the catalyst is typically between about 30 and 300 microns in major dimension. In fixed bed reactors, the catalyst may be in any suitable configuration including spheres, pellets, cylinders, monoliths, etc., and the size and shape may be influenced by pressure drop considerations for the gases passing through the bed. Often, the catalyst is at least about 0.2 centimeter, say, about 0.5 to 2 centimeters, in major dimension. Monolithic catalysts, which may comprise a support having the catalytically active component thereon or which may be homogeneous, can be sized to fit the reactor volume.

The process may be conducted in the presence of a selectivity-enhancing amount of metal component provided as a vapor phase component as described in copending patent application. Serial No. (D-16270), filed on even date herewith. When provided in the vapor phase, the metal component is introduced intermittently or continuously.

Often the amount of volatilized metal component is at least about 0.001 picogram, say about 0.005 picogram to 10000 or more milligrams per cubic meter of alkane in the feed (determined at standard temperature and pressure ("STP")).

It is not essential, and indeed in most instances it is not the case, that the volatilized metal component has a boiling point below the reaction temperature. Most convenient volatilized metal components have boiling points much higher than the reaction temperature under reaction conditions. However, the volatilized

6

metal component should have a vapor pressure under the reaction conditions sufficient to provide the sought amount of volatilized metal component to achieve the sought selectivity enhancement. Accordingly, the volatilized metal components are molten or near to becoming molten (e.g., within $100^\circ$ C) at the reaction temperature. The melting points of some volatilized metal components are set forth in Table I.

TABLE I

| Volatilized Component | Approximate Melting Point ($^\circ$C) |
|---|---|
| Sodium Chloride | 801 |
| Potassium Chloride | 770 |
| Sodium Bromide | 758 |
| Barium Iodide | 740 |
| Potassium Bromide | 730 |
| Rubidium Chloride | 718 |
| Potassium Iodide | 686 |
| Sodium Iodide | 653 |
| Cesium Chloride | 645 |
| Cesium Iodide | 612 |
| Lithium Chloride | 605 |
| Potassium Hydroxide | 405 |
| Sodium Hydroxide | 318 |

The preferred volatilized metal components are salts. Salts such as nitrates may have explosive characteristics at the reaction temperatures. Thus, these salts and others which may adversely decompose or oxidize are generally avoided. The volatilized metal component, however, may be added in the form of an oxide, hydroxide or salt and be converted to another compound under the reaction conditions. In general, the preferred salts are halides, especially chlorides, bromides or iodides.

The introduction of the volatilized metal component into the reaction zone may be by any convenient means. Advantageously, the volatilized metal component is relatively uniformly distributed as it passes through the reaction zone. The introduction may be, for instance, by adding a stream of volatilized metal component in the vapor form to the reaction zone or to the feed stream. Since most metal components are not gases under the reaction conditions, the volatilized metal components must enter the vapor phase through sublimation or the effects of partial pressure over the metal component in the liquid state. Hence, it is often desirable to pass, at an elevated temperature (e.g., $400^\circ$ to $1000^\circ$ C, say, $500^\circ$ to $850^\circ$ C), all or a portion of the feed gas over the metal component to volatilize a desired amount of the metal component. Since oxidation reactions can occur at these elevated temperatures, frequently, the metal component is contacted with either an alkane-containing stream having an essential absence of oxygen or a diluent gas or an oxygen-containing stream having an essential absence of alkane. The stream can be admixed with the remaining portion of the feed gases (for a continuous process).

In the event that the volatilized metal component solidifies, coalesces or is adsorbed in the reaction zone, a build-up of the metal component may occur. In many instances, the build-up is not unduly deleterious; however, if it adversely affects the performance of the reaction, temporary cessation of the introduction of the volatilized metal component may be indicated.

The volatilized metal component is capable of inhibiting vapor phase alkane conversion reactions. The volatilized metal component often comprises a metal which is at least as basic or electrophilic as lithium, and preferably, the metal is more basic or electrophilic than lithium. Frequently the volatilized metal component comprises at least one alkali metal or alkaline earth metal compound.

The halogen component is present in the reaction zone during the process. The halogen component is believed to be contained at least in the vapor space of the reactor. It is believed that the halogen component can be generated from a halogen-containing material on the catalyst. For instance, catalysts containing barium chloride or strontium chloride, at least when initially operated are believed to yield a chlorine-contaning component to the vapor phase. However, for long term operation and more stable performance of the catalyst, the addition of halogen component as a vapor phase additive is preferred. It may be added intermittently or continuously. The halogen component may be provided as a solid, liquid or vapor when added. The halogen component may be halogen, e.g., chlorine, bromine or iodine, or a halogen-containing compound. The halogen-containing compounds (chlorine, bromine and iodine-containing compound) may be inorganic or organic such as hydrogen halide, carbon tetrahalide, methylene halide, methyl dihalide, methyl trihalide, ethyl halide, ethyl dihalide, ethyl trihalide, ethyl tetrahalide, vinyl halide, sulfonyl chloride, phosphonyl chloride, etc. Often, the organic halides have from 1 to 3 halogen atoms and 1 to 3 carbon atoms. The amount of halogen component which can be added to the process, can vary; however, the amount added should be sufficient to provide the desired yield of higher hydrocarbon and the sought ethylene to ethane mole ratio. With either too little or too much halogen component addition, the catalyst performance will be adversely effected. Most frequently, if too little or too much halogen component has been added, good performance can be achieved by altering the rate of halogen component addition.

The amount of halogen component to be added for a given catalyst system will depend, inter alia , on the nature of the catalyst. Moreover, the optimum amount may change with the use of the catalyst.

Also, the type of halogen being added will influence the performance of the reaction system. In general, a process using a bromine compound as the halogen will provide a higher ratio of ethylene to ethane than a similar process using chlorine compound as the halogen. Within these guidelines, the amount of continuous vapor phase addition of the halogen component is often within the range of 0.1 to 5000, say, 1 to 1000, parts per million by volume based on the volume of feed to the reaction zone.

The following examples are provided by way of illustration of the invention and are not in limitation thereof. All parts and percentages of solids are by weight and of liquids and gases are by volume unless otherwise noted or clear from the context.

<div align="center">Example 1</div>

The following catalysts are prepared using the general procedures given below.

Procedure A: The selected alkaline earth metal component is dissolved in just enough deionized water at room temperature to wet the alpha-alumina support (The Norton Company, Akron, Ohio, SA-5402 30-60 mesh used for all catalysts). The alpha-alumina support is added to the solution and mixed to assure total uptake of the liquid and uniform impregnation of the alumina by incipient wetness. The alkaline earth metal component and alumina support are provided in relative amounts to each other to provide the desired loading of alkaline earth metal component on the catalyst. The material is then dried in a vacuum oven at about 110 to 130 °C. Drying is usually complete in 4 to 6 hours; however drying overnight does not appear to affect performance. The catalyst is tested without further treatment.

Procedure B: The selected insoluble alkaline earth metal component and the alpha-alumina support are added to 50 to 100 milliliters of deionized water at room temperature. The alkaline earth metal component and alpha-alumina support are provided in relative amounts to each other to provide the desired loading of alkaline earth metal component on the catalyst. The mixture is constantly stirred at about 80 to 90 °C until a thick paste remains. The paste is then dried in a vacuum oven at about 110 to 130 °C. Drying is usually complete in 4 to 6 hours; however drying overnight does not appear to affect performance. The catalyst is tested without further treatment.

Procedure C: The selected metal component is dissolved in just enough deionized water at room temperature to wet the alkaline earth metal component/alpha-alumina catalyst prepared using procedure A or B. In the event that the metal component is not sufficiently soluble to form a solution, concentrated hydrochloric acid is added dropwise until the component dissolves. The alkaline earth metal component/alpha-alumina catalyst is added to the solution and mixed to assure total uptake of the liquid and uniform impregnation of the alumina by incipient wetness. The metal component and alkaline earth metal component/alpha-alumina catalyst are provided in relative amounts to each other to provide the desired loading of metal component on the catalyst. The material is then dried in a vacuum oven at about 110 to 130 °C. Drying is usually complete in 4 to 6 hours; however drying overnight does not appear to affect performance. The catalyst is tested without further treatment.

Procedure D: The selected metal component is dissolved in just enough deionized water at room temperature to wet the alpha-alumina support. The alpha-alumina support is added to the solution and mixed to assure total uptake of the liquid and uniform wetting of the alumina. The metal component and alpha-alumina support are provided in relative amounts to each other to provide the desired loading of metal component on the catalyst. The material is then dried in a vacuum oven at about 110 to 130 °C. Drying is usually complete in 4 to 6 hours; however drying overnight does not appear to affect performance. The catalyst is tested without further treatment.

Procedure E: Procedure C is followed except that methanol and/or ethanol are used to dissolve the selected metal component

TABLE II

| Catalyst | Procedure | Dopant | Wt. % Based on Total Catalyst | Base Catalyst |
|---|---|---|---|---|
| A | A | None | --- | 3.0 wt% $SrCl_2$/alumina |
| B | A & C | $Co(NO_3)_2$ | 0.82 | 6.2 wt% $SrCl_2$/alumina |
| C | A & C | NiO | 0.34 | 6.2 wt% $SrCl_2$/alumina |
| D | A & C | $FeCl_2$ | 0.56 | 6.2 wt% $SrCl_2$/alumina |
| E | A & C | $Cu(NO_3)_2$ | 0.53 | 6.2 wt% $SrCl_2$/alumina |
| F | D | $Co(NO_3)_2$ | 0.79 | alumina |
| G | A & C | $Co(NO_3)_2$ | 0.81 | 6.9 wt% $BaCl_2$/alumina |
| H | B & C | $Co(NO_3)_2$ | 0.82 | 5.8 wt% $SrCO_3$/alumina |
| I | A & C | $RhCl_3$ | 0.91 | 11.6 wt% $SrCl_2$/alumina |
| J | A & E | $Pd(NO_3)_2$ | 0.66 | 11.6 wt% $SrCl_2$/alumina |

The following examples are conducted using the equipment described below. A quartz reactor is used which comprises a 1.5 centimeter (inside diameter) quartz tube about 55.9 centimeters in length with quartz "O"-ring joints at each end. At the bottom, a quartz effluent tube extends radially outward. Axially within the reactor tube is another quartz tube (1.3 centimeters outside diameter (1.1 centimeters inside diameter)) extending from the bottom (effluent end) of the reactor for about 28 centimeters. This tube is terminated with a joined 5 centimeters tube axially positioned thereon having an outside diameter of 0.5 centimeter and inside diameter of 0.3 centimeter. The annular region around this thinner tube ("annular reactor portion") receives the catalyst. These inner tubes form a thermocouple well. The thermocouple well extends 33 centimeters into the reactor from the bottom of the tube. The reactor is encased in a Lindberg oven for the mid-31 centimeters of its length. The incoming and exiting lines from the reactor are capable of being sampled by gas chromotography.

The catalyst bed is formed in the reactor by providing 20 to 40 mesh (U.S. Sieve Series) quartz chips around the larger diameter section of the thermocouple well, placing quartz wool over the chips (1 centimeter), forming the bed of catalysts (3 to 5 grams) wherein the catalyst particles have an average size of about 250 to 600 microns and then placing glass wool over the catalyst (1 centimeter) and either more quartz chips on the glass wool or a combination of an axially extending 1.3 centimeters outside diameter quartz solid rod with the quartz chips in the annular zone around the solid rod, to fill the upper portion of the reactor tube.

In the general operating procedures the reactor is flushed with nitrogen while heating to about reaction temperature. The reactant stream is fed and the reactor is brought to the desired temperature. periodic analyses of the gases are conducted (usually at intervals between one and two hours). The reactor pressure is about 5 pounds per square inch gauge (135 kPa absolute) and the feed usually contains $CH_4/O_2/N_2$ in a mole ratio of about 2/1/20. In the following Tables =/-designates the ethylene to ethane mole ratio, and time is the time that the catalyst is on stream. "$CH_4$ Conv." is the total percent of methane reacted based on the amount of methane in the product gas. "$C_2$ Sel." is the mole percent of carbon converted to ethylene and ethane compared to the total moles of carbon in the observed products and "$C_3$ Sel." is the mole percent of carbon converted to propylene and propane. "$C_2$ yield is the $CH_4$ Conv. times $C_2$ Sel./100. The gas hourly space velocity is based on the volumetric flow rate of the feed at ambient temperature and pressure per volume of the reactor occupied by the catalyst.

Example 2 (Comparative)

The comparison case performance of catalyst A using the equipment and procedures described previously is presented in Table III. Reactant feed gas ratio of $CH_4/O_2/N_2$ is 2.2/1/20.

TABLE III

| Temp. °C. | CH₄ Conv. % | C₂ Sel. % | C₃ Sel. % | C₂ Yield % | =/-Ratio molar | Time Hr. | GHSV Hr⁻¹ |
|---|---|---|---|---|---|---|---|
| 650 | 7.1 | 61 | 1.2 | 4.3 | 0.6 | 1 | 1500 |
| 650 | 7.5 | 61 | 3.0 | 4.5 | 0.6 | 3 | 1500 |
| 650 | 7.6 | 62 | 1.1 | 4.6 | 0.6 | 7 | 1500 |
| 650 | 5.0 | 57 | 0.8 | 2.9 | 0.4 | 15 | 1500 |
| 670 | 6.5 | 62 | 1.0 | 4.1 | 0.6 | 20 | 1500 |
| 670 | 4.7 | 59 | 0.9 | 2.8 | 0.5 | 22 | 1500 |

Example 3

The performance of catalyst 9 using the equipment and procedures described previously is presented in Tables IV and V. The results show the effect of adding cobalt to catalysts similar to catalyst A. Reactant feed gas ratio of $CH_4/O_2/N_2$ is 2.2/1/20.

TABLE IV

| Temp. °C. | CH₄ Conv. % | C₂ Sel. % | C₃ Sel. % | C₂ Yield % | =/-Ratio molar | Time Hr. | GHSV Hr⁻¹ |
|---|---|---|---|---|---|---|---|
| 650 | 33 | 65 | 3.3 | 20.7 | 5.2 | 1 | 1500 |
| 650 | 24 | 66 | 3.2 | 15.4 | 2.3 | 3 | 1500 |
| 650 | 21 | 67 | 3.2 | 13.7 | 1.9 | 11 | 1500 |
| 650 | 20 | 68 | 3.8 | 13.2 | 1.7 | 19 | 1500 |
| 670 | 29 | 69 | 3.9 | 18.8 | 3.0 | 21 | 1500 |
| 670 | 25 | 69 | 3.8 | 16.9 | 3.0 | 23 | 1500 |
| 670 | 22 | 67 | 3.0 | 14.1 | 1.7 | 31 | 1500 |
| 670 | 20 | 56 | 2.9 | 10.6 | 1.2 | 35 | 1500 |

TABLE V

| Temp. °C. | CH₄ Conv. % | C₂ Sel. % | C₃ Sel. % | C₂ Yield % | =/-Ratio molar | Time Hr. | GHSV Hr⁻¹ |
|---|---|---|---|---|---|---|---|
| 600 | 17 | 44 | 1.3 | 7.7 | 2.4 | 1 | 612 |
| 600 | 13 | 44 | --- | 6.1 | 1.5 | 3 | 612 |
| 600 | 11 | 45 | 1.0 | 4.9 | 1.4 | 7 | 612 |
| 625 | 20 | 56 | 2.3 | 11.6 | 2.5 | 9 | 612 |
| 625 | 19 | 56 | 2.2 | 10.6 | 2.2 | 13 | 612 |
| 625 | 16 | 58 | 2.1 | 9.6 | 1.8 | 21 | 612 |
| 650 | 27 | 61 | 3.2 | 17.5 | 4.1 | 23 | 612 |
| 650 | 29 | 65 | 3.1 | 19.5 | 4.5 | 27 | |
| 650 | 27 | 64 | 4.8 | 17.9 | 3.5 | 33 | |
| 650 | 24 | 67 | 3.2 | 16.2 | 3.0 | 45 | |

Example 4

The performance of catalyst C using the equipment and procedures described previously is presented in Table VI. The results show the effect of adding nickel to catalysts similar to catalyst A. Reactant feed gas ratio of $CH_4/O_2/N_2$ is 2.2/1/20.

TABLE VI

| Temp. °C. | $CH_4$ Conv. % | $C_2$ Sel. % | $C_3$ Sel. % | $C_2$ Yield % | =/-Ratio molar | Time Hr. | GHSV $Hr^{-1}$ |
|---|---|---|---|---|---|---|---|
| 650 | 22 | 70 | 5.0 | 14.8 | 2.2 | 1 | 1670 |
| 650 | 19 | 70 | 3.6 | 12.6 | 1.8 | 3 | 1670 |
| 650 | 18 | 69 | 2.8 | 12.2 | 1.5 | 7 | 1670 |
| 650 | 14 | 67 | 2.5 | 9.4 | 1.3 | 15 | 1670 |
| 670 | 20 | 70 | 3.4 | 13.7 | 2.3 | 17 | 1670 |
| 670 | 20 | 73 | 3.1 | 13.6 | 1.7 | 29 | 1670 |
| 670 | 17 | 70 | 3.7 | 11.4 | 1.3 | 39 | 1670 |

Example 5 (Comparative)

The performance of catalyst D using the equipment and procedures described previously is presented in Table VII. The results show the effect of adding iron to catalysts similar to catalyst A. Reactant feed gas ratio of $CH_4/O_2/N_2$ is 2.2/1/20.

TABLE VII

| Temp. °C. | $CH_4$ Conv. % | $C_2$ Sel. % | $C_3$ Sel. % | $C_2$ Yield % | =/-Ratio molar | Time Hr. | GHSV $Hr^{-1}$ |
|---|---|---|---|---|---|---|---|
| 650 | 6.9 | 16 | 0.0 | 1.2 | 0.7 | 1 | 1580 |
| 650 | 5.1 | 5.3 | 0.2 | 0.3 | 0.4 | 3 | 1580 |
| 650 | 12 | 2.0 | 0.0 | 0.3 | 0.4 | 11 | 1580 |
| 650 | 15 | 1.0 | 0.0 | 0.2 | 0.6 | 39 | 1580 |
| 670 | 19 | 1.4 | 0.0 | 0.3 | 0.6 | 41 | 1580 |
| 670 | 18 | 1.4 | 0.0 | 0.3 | 0.6 | 45 | 1580 |
| 750 | 17 | 1.4 | 0.0 | 0.3 | 0.6 | 47 | 1580 |
| 750 | 21 | 2.5 | 0.0 | 0.5 | 0.6 | 63 | 1580 |

Example 6 (Comparative)

The performance of catalyst E using the equipment and procedures described previously is presented in Table VIII. The results show the effect of adding copper to catalysts similar to catalyst A. Reactant feed gas ratio of $CH_4/O_2/N_2$ is 2.2/1/20.

TABLE VIII

| Temp. °C. | CH₄ Conv. % | C₂ Sel. % | C₃ Sel. % | C₂ Yield % | =/-Ratio molar | Time Hr. | GHSV Hr⁻¹ |
|---|---|---|---|---|---|---|---|
| 650 | 1.0 | 0.6 | 4.0 | 0.0 | 0.5 | 1 | 1460 |
| 650 | 3.7 | 0.2 | 0.3 | 0.0 | 1.6 | 9 | 1460 |
| 650 | 3.6 | 0.1 | 0.0 | 0.0 | --- | 41 | 1460 |
| 670 | 7.3 | 0.2 | 0.0 | 0.0 | 1.7 | 43 | 1460 |
| 670 | 8.5 | 0.2 | 0.0 | 0.0 | 1.4 | 47 | 1460 |
| 750 | 18 | 1.3 | 0.0 | 0.2 | 0.3 | 49 | 1460 |
| 750 | 21 | 4.7 | 0.0 | 1.0 | 0.8 | 59 | 1460 |

Example 7

The performance of catalyst I using the equipment and procedures described previously is presented in Table IX. The results show the effect of adding rhodium to catalysts similar to catalyst A. Reactant feed gas ratio of $CH_2/O_2/N_2$ is 2.1/1/19.

TABLE IX

| Temp. °C. | CH₄ Conv. % | C₂ Sel. % | C₃ Sel. % | C₂ Yield % | =/-Ratio molar | Time Hr. | GHSV Hr⁻¹ |
|---|---|---|---|---|---|---|---|
| 600 | 26 | 45 | 2.2 | 11.8 | 3.7 | 3 | 790 |
| 600 | 18 | 38 | 1.4 | 6.6 | 1.9 | 9 | 790 |
| 600 | 14 | 32 | 0.9 | 4.6 | 1.4 | 15 | 790 |
| 625 | 23 | 43 | 1.9 | 9.7 | 2.6 | 17 | 790 |
| 625 | 22 | 40 | 1.1 | 8.8 | 2.4 | 23 | 790 |
| 650 | 36 | 51 | 2.0 | 18.5 | 4.4 | 25 | 790 |
| 650 | 34 | 47 | 3.1 | 16.0 | 4.3 | 29 | 790 |
| 650 | 33 | 48 | 3.6 | 16.2 | 3.6 | 37 | 790 |

Example 8 (Comparative)

The performance of catalyst J using the equipment and procedures described previously is presented in Table X. The results show the effect of adding palladium to catalysts similar to catalyst A. Reactant feed gas ratio of $CH_4/O_2/N_2$ is 2.0/1/18.

TABLE X

| Temp. °C. | CH₄ Conv. % | C₂ Sel. % | C₃ Sel. % | C₂ Yield % | =/-Ratio molar | Time Hr. | GHSV Hr⁻¹ |
|---|---|---|---|---|---|---|---|
| 600 | 8.0 | 14 | 13 | 1.2 | 0.6 | 45 | 750 |
| 600 | 8.2 | 14 | 12 | 1.2 | 0.5 | 49 | 750 |
| 600 | 7.8 | 14 | 12 | 1.1 | 0.5 | 53 | 750 |
| 650 | 24 | 32 | 10 | 7.9 | 2.4 | 55 | 750 |
| 650 | 18 | 30 | 12 | 5.7 | 1.7 | 59 | 750 |
| 650 | 18 | 19 | 8.0 | 3.4 | 0.9 | 65 | 750 |
| 675 | 24 | 18 | 0.4 | 4.4 | 0.9 | 67 | 750 |
| 675 | 23 | 9.8 | 0.1 | 2.3 | 0.5 | 69 | 750 |

Example 9 (Comparative)

The performance of catalyst F using the equipment and procedures described previously is presented in Tables XI. The results show the effect of the absence of $SrCl_2$ to catalysts similar to catalyst B. Reactant feed gas ratio of $CH_4/O_2/N_2$ is 2.0/1/18.

TABLE XI

| Temp. °C. | CH₄ Conv. % | C₂ Sel. % | C₃ Sel. % | C₂ Yield % | =/-Ratio molar | Time Hr. | GHSV Hr⁻¹ |
|---|---|---|---|---|---|---|---|
| 600 | 19 | 3.0 | 0.0 | 0.6 | 0.8 | 1 | 590 |
| 600 | 18 | 0.9 | 0.0 | 0.2 | 1.8 | 5 | 590 |
| 600 | 16 | 0.3 | 0.0 | 0.1 | 0.4 | 11 | 590 |
| 625 | 17 | 0.1 | 0.0 | 0.0 | 0.2 | 22 | 590 |
| 650 | 19 | 0.4 | 0.0 | 0.1 | 0.6 | 34 | 590 |

Example 10

The performance of catalyst G using the equipment and procedures described previously is presented in Tables XII. The results show the effect of the replacing $SrCl_2$ with $BaCl_2$ in catalysts similar to catalyst B. Reactant feed gas ratio of $CH_4/O_2/N_2$ is 2.2/1/20.

TABLE XII

| Temp. °C. | CH₄ Conv. % | C₂ Sel. % | C₃ Sel. % | C₂ Yield % | =/-Ratio molar | Time Hr. | GHSV Hr⁻¹ |
|---|---|---|---|---|---|---|---|
| 600 | 6.2 | 8.8 | 0.0 | 0.6 | 0.7 | 1 | 714 |
| 600 | 4.1 | 11 | 0.0 | 0.5 | 0.8 | 7 | 714 |
| 625 | 9.4 | 9.8 | 0.0 | 0.9 | --- | 9 | 714 |
| 625 | 7.1 | 11 | 0.0 | 0.8 | --- | 19 | 714 |
| 650 | 15 | 41 | 0.7 | 6.3 | 1.1 | 21 | 714 |
| 650 | 16 | 49 | 0.9 | 8.0 | 1.1 | 29 | 714 |
| 650 | 16 | 47 | 0.8 | 7.8 | 1.1 | 37 | 714 |
| 670 | 23 | 57 | 1.4 | 13.9 | 1.1 | 41 | 714 |
| 670 | 24 | 57 | 1.3 | 14.1 | 1.1 | 47 | 714 |

Example 11

The performance of catalyst H using the equipment and procedure described previously is presented in Table XIII. The results show the effect of the replacing $SrCl_2$ with $SrCO_3$ in catalysts similar to catalyst B. Reactant feed gas ratio of $CH_4/O_2/N_2$ is 2.2/1/20.

TABLE XIII

| Temp. °C. | CH₄ Conv. % | C₂ Sel. % | C₃ Sel. % | C₂ Yield % | =/-Ratio molar | Time Hr. | GHSV Hr⁻¹ |
|---|---|---|---|---|---|---|---|
| 600 | 20 | 3.4 | 0.0 | 0.7 | 0.8 | 3 | 714 |
| 600 | 16 | 0.5 | 0.0 | 0.1 | 0.7 | 9 | 714 |
| 625 | 20 | 3.2 | 0.0 | 0.7 | 0.9 | 17 | 714 |
| 625 | 21 | 2.7 | 0.0 | 0.6 | 0.8 | 21 | 714 |
| 650 | 22 | 2.8 | 0.0 | 0.6 | 0.9 | 23 | 714 |
| 650 | 22 | 0.6 | 0.0 | 0.1 | 0.4 | 39 | 714 |

Example 12

The performance of catalyst H using the equipment and procedures described previously is presented in Table XIV. The results show the effect of ethyl chloride (ECl) in the feed stream on catalyst H. Reactant feed gas ratio of $CH_4/O_2/N_2$ is 2.2/1/20 with an ethyl chloride concentration of 100 ppm.

TABLE XIV

| Temp. °C. | CH₄ Conv. % | C₂Sel. % | C₃Sel. % | C₂Yield % | =/-Ratio molar | Time Hr. | GHSV Hr⁻¹ |
|---|---|---|---|---|---|---|---|
| 650 | 13 | 8.5 | 0.0 | 1.1 | 0.7 | 1 | 880 |
| 650 | 13 | 12 | 0.0 | 1.6 | 0.8 | 5 | 880 |
| 650 | 12 | 18 | 0.2 | 2.2 | 0.9 | 13 | 880 |
| 650 | 12 | 25 | 0.4 | 3.0 | 1.1 | 17 | 880 |
| 700 | 23 | 54 | 2.3 | 12.5 | 2.0 | 21 | 880 |
| 700 | 18 | 51 | 3.3 | 9.5 | 1.7 | 27 | 880 |
| 700 | 14 | 43 | 1.3 | 6.4 | 1.6 | 39 | 880 |
| 650 | 6.0 | 37 | 0.9 | 2.3 | 1.0 | 43 | 880 |
| 650 | 6.1 | 39 | 0.7 | 2.4 | 1.0 | 51 | 880 |

Example 13 (comparative)

The performance of catalyst A using the equipment and procedures described previously is presented in Table XV. The results show the effect of ethyl chloride (ECl) in the feed stream on catalyst A. Reactant feed gas ratio of CH₄/O₂/N₂ is 2.2/1/20 with an ethyl chloride concentration of 50 ppm and 3.4/1/19 for an ethyl chloride concentration of 100 ppm.

TABLE XV

| Temp. °C. | ECl ppmv | CH₄ Conv. % | C₂Sel. % | C₂Yield % | =/-Ratio molar | Time Hr. | GHSV Hr⁻¹ |
|---|---|---|---|---|---|---|---|
| 650 | 50 | 8.9 | 67 | 6.1 | 1.6 | 1 | 600 |
| 650 | 50 | 7.5 | 66 | 5.0 | 1.4 | 7 | 600 |
| 650 | 50 | 7.5 | 66 | 5.0 | 1.4 | 23 | 600 |
| 650 | 50 | 3.9 | 66 | 2.6 | 0.9 | 41 | 600 |
| 650 | 100 | 4.8 | 72 | 3.4 | 0.6 | 73 | 600 |
| 650 | 100 | 4.9 | 74 | 3.6 | 0.6 | 83 | 600 |
| 750 | 100 | 12 | 47 | 5.8 | 1.6 | 129 | 600 |
| 750 | 100 | 12 | 38 | 4.7 | 1.6 | 139 | 600 |
| 750 | 100 | 12 | 37 | 4.5 | 1.6 | 143 | 600 |
| 750 | 100 | 13 | 37 | 4.8 | 1.8 | 149 | 480 |

Example 14

The performance of catalyst B using the equipment and procedures described previously is presented in Table XVI. The results show the effect of ethyl chloride (ECl) in the feed stream on catalyst B. Reactant feed gas ratio of CH₄/O₂/N₂ is 2.4/1/20 with an ethyl chloride concentration of 50 ppm and 3.4/1/19 for an ethyl chloride concentration of 100 ppm. ethane ratio.

TABLE XVI

| Temp. °C. | ECl ppmv | CH₄ Conv. % | C₂ Sel. % | C₂ Yield % | =/-Ratio molar | Time Hr. | GHSV Hr⁻¹ |
|---|---|---|---|---|---|---|---|
| 643 | 50 | 26 | 55 | 14.6 | 6.2 | 1 | 760 |
| 643 | 50 | 22 | 62 | 13.9 | 4.1 | 7 | 760 |
| 643 | 50 | 19 | 66 | 12.9 | 3.0 | 23 | 760 |
| 643 | 50 | 16 | 64 | 11.2 | 2.5 | 41 | 760 |
| 643 | 100 | 16 | 69 | 10.8 | 2.2 | 73 | 760 |
| 643 | 100 | 16 | 72 | 11.2 | 2.2 | 81 | 760 |
| 643 | 100 | 15 | 65 | 10.1 | 1.9 | 129 | 760 |
| 643 | 100 | 15 | 62 | 9.2 | 1.9 | 139 | 760 |
| 643 | 100 | 14 | 61 | 8.8 | 1.8 | 143 | 760 |
| 643 | 100 | 19 | 60 | 11.6 | 2.4 | 150 | 490 |

## Claims

1. A process for oxidation coupling of alkane of 1 to 3 carbon atoms to heavier hydrocarbon comprising feeding the alkane and reactive oxygen-containing material to a reaction zone containing a catalytically effective amount of oxidative coupling catalyst, said oxidation coupling catalyst being a supported catalyst comprising a catalytically active amount of at least one alkali metal or alkaline earth metal compound sufficient to enhance selectivity to heavier hydrocarbon and up to about 2 weight percent of cobalt compound and/or nickel compound and/or rhodium compound in an amount sufficient to enhance oxidative coupling activity; maintaining the reaction zone under oxidative coupling conditions comprising a bulk temperature of less than about 700°C, to convert at least a portion of the alkane to heavier hydrocarbons said reaction zone containing halogen-containing component in an amount sufficient to enhance at least one of conversion of alkane and selectivity to higher hydrocarbon, and withdrawing from the reaction zone an effluent containing heavier hydrocarbons produced in the reaction zone.

2. The process of claim 1 wherein the reactive oxygen-containing material comprises oxygen.

3. The process of claim 2 wherein the catalyst comprises Group IIA metal compound.

4. The process of claim 3 wherein the catalyst comprises at least one of barium compound and strontium compound.

5. The process of claim 4 wherein at least one barium and/or strontium compound comprises at least one of oxide, peroxide, hydroxide, carbonate, chloride, bromide and iodide.

6. The process of claim 4 wherein barium and/or strontium compound comprises 1 to 20 weight percent of the catalyst.

7. The process of claim 2 wherein the halogen-containing component comprises halogen-containing vapor phase additive having at least one of hydrogen halide, organic halide of 1 to 3 carbon atoms and halogen, wherein the halide or halogen is at least one of chlorine, bromine and iodine.

8. The process of claim 7 wherein the halogen-containing vapor phase additive is provided in an amount of about 1 to 1500 ppm based on the feed to the reaction zone.

9. The process of claim 7 wherein the oxidative coupling conditions comprise a temperature between about 550°C and 675°C and a gas hourly space velocity of between about 500 and 15000 reciprocal hours.

10. The process of claim 9 wherein the mole ratio of alkane to oxygen atom is about 1:1 to 20:1.

11. The process of claim 10 wherein the halogen-containing vapor phase additive comprises chlorine.

12. The process of claim 3 wherein the catalyst comprises cobalt compound.

13. The process of claim 12 wherein the halogen-containing component comprises halogen-containing vapor phase additive having at least one of hydrogen halide, organic halide of 1 to 3 carbon atoms and halogen, wherein the halide or halogen is at least one of chlorine, bromine and iodine.

14. The process of claim 3 wherein the support comprises at least one of catalytically active Group IA or Group IIA metal compound.

15. The process of claim 3 wherein the support comprises alumina.

16. The process of claim 2 wherein the total cobalt and/or nickel and/or rhodium compound is present in an amount of between 0.01 and 1.0 weight percent based (calculated as the metal) on the total weight of the

catalyst.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 110, no. 5, 27th February 1989, page 602, abstract no. 75024j, Columbus, Ohio, US; & SU-A-1 395 620 (MAMEDALIEV, Yu. G., INSTITUTE OF PETROCHEMICAL PROCESSES) 15-05-1988 --- | 1 | C 07 C 2/84 |
| X | US-A-4 465 893 (OLAH) * Claims * --- | 1,7 | |
| X | DE-A-3 534 530 (M. BAERNS) * Claims * . --- | 1-5 | |
| X | US-A-4 654 459 (SOFRANKO) * Claims * --- | 1-5 | |
| X | CHEMICAL ABSTRACTS, vol. 108, no. 21, May 1988, page 648, abstract no. 186163g, Columbus, Ohio, US; & JP-A-62 267 243 (IDEMITSU KOSAN CO., LTD) 19-11-1987 --- | 1-5 | |
| A | DE-A-3 503 664 (AKZO) ----- | | |

TECHNICAL FIELDS SEARCHED (Int. Cl.5)

C 07 C 2/00

The present search report has been drawn up for all claims

| Place of search | Date of completion of search | Examiner |
|---|---|---|
| The Hague | 28 November 90 | VAN GEYT J.J.A. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document